# EUROPÄISCHE PATENTSCHRIFT

(11) **EP 3 478 195 B1**
(45) Veröffentlichungstag und Bekanntmachung des Hinweises auf die Patenterteilung: **01.01.2020**
(21) Anmeldenummer: 17736605.1
(22) Anmeldetag: 28.06.2017
(51) Int. Cl.: A61B 17/70, A61B 17/88, A61F 2/44, A61F 2/30

(54) **EXPANDIERBARES ZWISCHENWIRBELIMPLANTAT**
EXPANDABLE INTERVERTEBRAL IMPLANT
IMPLANT INTERVERTÉBRAL EXPANSIBLE

(30) Priorität: 29.06.2016 DE 102016111886
(43) Veröffentlichungstag der Anmeldung: 08.05.2019
(73) Patentinhaber: Silony Medical International AG, 8500 Frauenfeld (CH)
(72) Erfinder: MAYER, Michael Heinz, 82166 Graefelfing (DE); STADLBAUER, Gunter, 78262 Gailingen (DE); HEUER, Frank, 70794 Filderstadt (DE)
(74) Vertreter: DREISS Patentanwälte PartG mbB
(86) Internationale Anmeldenummer: PCT/EP2017/066003
(87) Internationale Veröffentlichungsnummer: WO 2018/002145

(56) Entgegenhaltungen:
- US-A1- 2007 168 031
- US-A1- 2007 276 337
- US-A1- 2007 288 095
- US-A1- 2012 016 371

## Beschreibung

Die Erfindung betrifft ein expandierbares Zwischenwirbelimplantat für den Bandscheibenersatz, insbesondere im thorakalen und lumbalen Wirbelsäulenbereich. Als Bandscheibenersatz wurden bislang sogenannte "Cages" verwendet, die in verschiedener Weise an die erwünschte Gestalt des Bandscheibenfachs angepasst werden können, indem die relative Position ihrer oberen und unteren Wandung zueinander durch verschiedene Mechanismen einstellbar ist. Patentdokumente US2012/0016371 und US2007/0276337 offenbaren expandierbare Zwischenwirbelimplantate entsprechend dem Oberbegriff der unabhängigen Ansprüche.

Der vorliegenden Erfindung liegt die Aufgabe zugrunde, ein expandieres Zwischenwirbelimplantat für den Bandscheibenersatz bereitzustellen, mit dem einerseits eine optimale Anpassung des Implantats an das auszufüllende Bandscheibenfach möglich ist und andererseits das Bandscheibenfach wie vom Chirurgen intendiert gestützt oder aufgeweitet werden kann und in diesem distrahierten Zustand dauerhaft stabilisiert werden kann.

Diese Aufgabe wird erfindungsgemäß gelöst durch ein expandierbares Zwischenwirbelimplantat mit den Merkmalen des Anspruchs 1 und durch ein expandierbares Zwischenwirbelimplantat mit den Merkmalen des Anspruchs 10.

Anspruch 1 schlägt vor ein expandierbares Zwischenwirbelimplantat für den Bandscheibenersatz, insbesondere im thorakalen und lumbalen Wirbelsäulenbereich, mit einem inneren Ballonelement und einem äußeren Ballonelement, wobei das innere und das äußere Ballonelement jeweils für sich mittels eines injizierbaren fluiden und aushärtbaren Mediums füllbar sind, wobei das äußere Ballonelement beim Einbringen in ein Bandscheibenfach in einem Zwischenwirbelbereich als Bandscheibenersatz ungefähr die Gestalt oder Teile der zuvor entfernten Bandscheibe einnehmen soll, und eine obere und eine untere Wandung umfasst, mittels derer es im injizierten Zustand gegen eine das Bandscheibenfach oben und unten begrenzende Seite der angrenzenden Wirbelkörper anlegbar ist, und wobei das innere Ballonelement eine anteriore oder posteriore oder parallele Bandscheibenfacherhöhung bewirkt, indem es im injizierten Zustand von innen gegen das äußere Ballonelement mittelbar oder unmittelbar stützend anliegt. Dieses Zwischenwirbelimplantat ist erfindungsgemäß weiter so ausgebildet, dass wenigstens eine zusätzliche Zuführleitung für das injizierbare fluide und aushärtbare Medium vorgesehen ist, die in der oberen und/oder unteren Wandung des äußeren Ballonelements nach außen mündet, so dass das injizierte Medium in angrenzende poröse Knochenbereiche eindringen kann. Erfindungsgemäß ist also das äußere Ballonelement entsprechend der zu ersetzenden Bandscheibe ausgebildet, es weist im befüllten oder injizierten Zustand eine scheibenförmige, der ungefähren Kontur der Bandscheibe folgende Gestalt auf, wobei das äußere Ballonelement im injizierten Zustand und je nach Positionierung und Befüllungszustand des inneren Ballonelements eine variierende vom Chirurgen individuell einstellbare Höhe in spinaler Richtung aufweist. Je nach Anordnung oder Positionierung des inneren Ballonelements innerhalb äußeren Ballonelements in anteriorer oder posteriorer Richtung und je nach Befüllung des inneren Ballonelements kann mittels des inneren Ballonelements eine anteriore oder posteriore oder parallele Bandscheibenfacherhöhung bewirkt werden, insbesondere entsprechend einer zu korrigierenden Lordose oder Kyphose oder auch Spinose. Dabei wird in der Operationssituation zunächst das Zwischenwirbelimplantat im zu distrahierenden Bandscheibenfach angeordnet, und dann wird das innere Ballonelement durch Injizieren des fluiden und aushärtbaren Mediums derart befüllt, dass es unten und oben gegen die Innenseite des äußeren Ballonelements unter Kraftausübung anliegt und hierdurch eine Erhöhung oder Veränderung des Bandscheibenfachs erreichen kann.

Wie bereits erwähnt, weist das äußere Ballonelement im injizierten, mit fluidem Medium ausgefüllten Zustand eine scheibenförmige Gestalt auf. Es erweist sich als vorteilhaft, wenn zwischen der oberen und unteren Wandung eine ungefähr streifenförmige in einer Umfangsrichtung erstreckte Seitenwandung vorgesehen ist, welche oben und unten in die obere bzw. untere Wandung anschließt.

Weiter erweist es sich als vorteilhaft, wenn das äußere und vorzugsweise auch das innere Ballonelement im injizierten Zustand eine Vorzugskontur einnimmt. Diese der grundsätzlichen Bandscheibenkontur entsprechende Vorzugskontur kann beispielsweise dadurch realisiert werden, dass Flachmaterialabschnitte entsprechend zugeschnitten und zur Bildung des Ballonelements aneinander gefügt werden. Es ist auch denkbar und vorteilhaft, wenn in Flachmaterialabschnitte Drähte, Lamellen, Fasern, Filamente oder hieraus gewobene Flachmaterialstrukturen eingebracht werden, welche die Ausbildung einer Vorzugskontur bewirken oder unterstützen. Beispielsweise können biegsame Drähte, wie Nitinol (Nickel-Titan-Legierung), entlang des Außenumfangs der oberen und unteren Wandungen eingebracht werden. Metallische Drähte haben den Vorteil, dass sie unter Röntgenkontrolle sichtbar sind. Es wäre auch denkbar, dass die in der Umfangsrichtung erstreckte Seitenwandung des äußeren Ballonelements eine höhere Steifigkeit als die obere oder untere Wandung aufweist, welche der Formstabilität dient.

Weiter erweist es sich als vorteilhaft, dass im Inneren des äußeren Ballonelements ein anterior angeordnetes und ein posterior angeordnetes inneres Ballonelement vorgesehen sind. Auf diese Weise kann durch Befüllen des anterior bzw. posterior angeordneten inneren Ballonelements auf sehr einfache Weise gezielt eine erwünschte Erhöhung des Bandscheibenfachs anterior oder posterior erreicht werden, insbesondere um eine Lordose oder Kyphose zu korrigieren. Alternativ wäre es denkbar, dass je nach zu erzielender Bandscheibenfachaufweitung ein Zwischenwirbelimplantat mit einem posterior angeordneten bzw. alternativ mit einem anterior angeordneten inneren Ballonelement verwendet wird. Es wäre auch denkbar, dass das innere Ballonelement in anteriorer bzw. posteriorer Richtung innerhalb des äußeren Ballonelements verschieblich positionierbar ist.

Es erweist sich weiter als vorteilhaft, wenn von dem inneren Ballonelement ein leitungsbildendes Verbindungselement nach außerhalb des äußeren Ballons führt, an welches ein Instrument zum Injizieren des fluiden und aushärtbaren Mediums lösbar ankoppelbar ist.

Dieses leitungsbildende Verbindungselement kann zugleich die Zuführleitung umfassen oder mit ihr kommunizieren.

Es erweist sich als vorteilhaft, wenn das mit dem inneren Ballonelement kommunizierende leitungsbildende Verbindungselement ein Ventil umfasst, welches bei von außen anliegendem Injektionsdruck öffnet und bei Wegnahme des von außen anliegenden Injektionsdrucks schließt. Dies bringt den Vorteil mit sich, dass das innere Ballonelement beim Befüllen des äußeren Ballonelements nicht zwingend unter Injektionsdruck gehalten werden muss, was die Implantation vereinfacht. In Weiterbildung dieses Erfindungsgedankens erweist es sich als vorteilhaft, wenn das Ventil dadurch öffenbar ist, dass ein Ventilkörper des Ventils von außen mittels einer Kanüle, durch die das fluide und aushärtbare Medium injizierbar ist, verdrängt wird. Wenn die Kanüle dann geringfügig zurückbewegt wird, so bewirkt der Fülldruck im Inneren des inneren Ballonelements, dass der Ventilkörper des Ventils wieder von innen gegen seinen Ventilsitz dichtend angelegt wird.

Nach einem weiteren Erfindungsgedanken von wesentlicher Bedeutung erweist es sich als vorteilhaft, dass das leitungsbildende Verbindungselement in einem Bereich zwischen einer Außenseite des inneren Ballonelements und einer Innenseite der äußeren Ballonelements eine Öffnung zum Injizieren des fluiden und aushärtbaren Mediums in das Innere des äußeren Ballonelements aufweist. Es ist also möglich, unter Verwendung ein und desselben leitungsbildenden Verbindungselements sowohl das innere Ballonelement als auch das äußere Ballonelement mit fluidem aushärtbarem Medium zu befüllen (injizieren) und dadurch eine Distraktion des Bandscheibenfachs herbeizuführen.

Hierbei erweist es sich als vorteilhaft, dass die Öffnung zum Injizieren des fluiden und aushärtbaren Mediums in das äußere Ballonelement beim Injizieren des fluiden und aushärtbaren Mediums in das innere Ballonelement verschließbar ist.

Dies kann in vorteilhafter Weise dadurch realisiert werden, dass die Öffnung zum Injizieren des fluiden und aushärtbaren Mediums in das äußere Ballonelement durch eine Kanüle, durch die das fluide und aushärtbare Medium injizierbar ist, verschließbar und freigebbar ist.

Weiter erweist es sich als vorteilhaft, wenn das leitungsbildende Verbindungselement gegenüber dem äußeren Ballonelement längenverstellbar ist, so dass das innere Ballonelement nach anterior oder posterior verlagerbar ist.

Es wäre auch denkbar, dass jedem Ballonelement ein leitungsbildendes Verbindungselement zugeordnet ist, an welches ein Instrument zum Injizieren des fluiden und aushärtbaren Mediums lösbar ankoppelbar ist.

Nach einem weiteren Erfindungsgedanken von wesentlicher Bedeutung erweist es sich als vorteilhaft, wenn das Zwischenwirbelimplantat mit einer Osteosynthesevorrichtung verbindbar ist, die an wenigstens einem angrenzenden Wirbelkörper fixierbar ist. Durch Verbindung oder Anbindung des Zwischenwirbelimplantats an eine Osteosynthesevorrichtung kann eine gemeinsam handhabbare Einrichtung geschaffen werden, durch die das Zwischenwirbelimplantat mittels der Osteosynthesevorrichtung gehalten und eingebracht werden kann. Diese Halteverbindung kann in vorteilhafter Weise dadurch realisiert werden, dass das Zwischenwirbelimplantat und die Osteosynthesevorrichtung mittels des leitungsbildenden Verbindungselements miteinander verbunden sind.

Bei dem injizierbaren fluiden und aushärtbaren Medium kann es sich in vorteilhafter Weise um PMMA (Knochenzement), Hydrogel, Silikon und/oder Calciumphosphat (CaP) und gegebenenfalls partikuläre Füllstoffe handeln.

Weiter erweist es sich als vorteilhaft, wenn das äußere Ballonelement eine äußere Beschichtung mit Titan oder Titanlegierung, mit Hydroxylapatit (HA), mit Polylactid (PLA), mit Calciumphosphat (CaP), mit BMP (Bone Morphogenetic Protein = knochenmorphogenetische Proteine), und/oder mit Silber oder einer Silberverbindung aufweist. Hierdurch kann eine stabilere bzw. integrative Verbindung zwischen dem äußeren Ballonelement und dem Knochen erreicht werden. Dies wird auf eine verbesserte biologische Interaktion zwischen dem Ballonelement und dem Knochengewebe zurückgeführt. Hierfür ist es auch denkbar, dass das äußere Ballonelement außen eine Nano-, Mikro- oder Makrostrukturierung aufweist, die beispielsweise durch Ätz- oder Laserverfahren hergestellt werden kann, oder außen spikeartige Vorsprünge aufweist, die sich in den angrenzenden Knochen eingraben und verankern können.

Es erweist sich auch als besonders vorteilhaft, wenn das äußere Ballonelement zumindest bereichsweise in der oberen und/oder unteren Wandung Öffnungen aufweist, die oberhalb eines Innendrucks für das injizierte Medium durchlässig werden, so dass das injizierte Medium die Öffnungen durchdringen und in angrenzende poröse Knochenbereiche eindringen kann.

Zu diesem Zweck erweist es sich auch als vorteilhaft, wenn wenigstens eine zusätzliche Zuführleitung für das injizierbare fluide und aushärtbare Medium vorgesehen ist, die in der oberen und/oder unteren Wandung des äußeren Ballonelements nach außen mündet, so dass das injizierte Medium in angrenzende poröse Knochenbereiche eindringen kann. Insbesondere kann das leitungsbildende Verbindungselement diese Zuführleitung umfassen oder mit ihr kommunizieren.

Es wird auch Schutz beansprucht für ein Instrument zum Befüllen des inneren oder äußeren Ballonelements und für eine Sachgesamtheit aus einem erfindungsgemäßen expandierbaren Zwischenwirbelimplantat und einem solchen Instrument zum Injizieren des fluiden und aushärtbaren Mediums in das innere und das äußere Ballonelement mit den Merkmalen der Ansprüche 13-15.

Durch das Verschieben der Kanüle des Instruments kann die Kanüle in eine Injektionsstellung in Bezug auf das innere oder das äußere Ballonelement gebracht werden. Beispielsweise vermag sie in einer Injektionsstellung für das innere Ballonelement das dort vorgesehene Ventil zu öffnen und in einer zurückgezogenen Position zu verschließen.

Es erweist sich auch als vorteilhaft, wenn die Kanüle in der äußeren Hülse in einer Längsrichtung begrenzt verschieblich ist, indem sie wenigstens einen radialen Vorsprung aufweist, der in einer von einem Griffelement der äußeren Hülse begrenzten Kammer gegen einen Axialanschlag oder vorzugsweise in beiden Richtungen gegen je einen Axialanschlag anlegbar ist. Auf diese Weise kann eine Injektionsposition eingenommen bzw. verlassen werden.

Weiter kann es sich als vorteilhaft erweisen, wenn die Kanüle des Instruments in der äußeren Hülse in einer Längsrichtung drehbar ist und eine seitliche Fluidaustrittsöffnung aufweist, die durch Drehung in eine fluchtende Anordnung mit der Öffnung in dem leitungsbildenden Verbindungselement bringbar ist, so dass durch Drehung der Kanüle eine Fluidkommunikation zum Infiltrieren des äußeren Ballonelements herstellbar und blockierbar ist. Somit kann die Öffnung zum Befüllen des äußeren Ballonelements freigegeben oder verschlossen werden.

Nach einer weiteren Ausführungsform der Erfindung ist vorgesehen, dass die Kanüle in der äußeren Hülse an Ihrem ballonseitigen Ende geschlossen ist und in einer Längsrichtung drehbar ist und eine oder mehrere seitliche Fluidaustrittsöffnung aufweist, die durch Drehung in eine fluchtende Anordnung mit wenigstens einer Öffnung in dem leitungsbildenden Verbindungselement bringbar ist, wobei diese wenigstens eine Öffnung mit dem Inneren eines Ballonelements kommuniziert, so dass durch Drehung der Kanüle eine Fluidkommunikation zum Infiltrieren eines oder mehrerer Ballonelemente herstellbar und blockierbar ist.

Die eingangs genannte Aufgabe wird aber auch durch ein Zwischenwirbelimplantat mit den Merkmalen des Anspruchs 21 gelöst. Bei diesem Implantat ist nicht zwingend ein zusätzliches inneres Ballonelement vorgesehen, sondern die erfindungsgemäße Ausbildung kann auch bei einem einzigen Ballonelement vorgesehen werden.

Weitere Merkmale, Einzelheiten und Vorteile der Erfindung ergeben sich aus den beigefügten Patentansprüchen und aus der zeichnerischen Darstellung und nachfolgenden Beschreibung verschiedener Ausführungsformen der Erfindung.

In der Zeichnung zeigt:
- Figur 1: eine perspektivische Ansicht eines Zwischenwirbelimplantats (nicht erfindungsgemäß), wobei auch innen liegende Bestandteile dargestellt sind;
- Figur 2: eine weitere Ausführungsform eines Zwischenwirbelimplantats (nicht erfindungsgemäß) mit einem Injektionsanschluss schräg/ventral;
- Figur 3: eine weitere Ausführungsform eines Zwischenwirbelimplantats (nicht erfindungsgemäß) mit zwei inneren Ballonelementen;
- Figur 4: eine schematische Darstellung eines Zwischenwirbelimplantats (nicht erfindungsgemäß) zur Verdeutlichung des Injektionsvorgangs des inneren Ballonelements;
- Figur 5: eine schematische Darstellung des Zwischenwirbelimplantats (nicht erfindungsgemäß) nach Figur 4 beim Befüllen des äußeren Ballonelements;
- Figur 6a: eine perspektivische Darstellung der Bestandteile einer Sachgesamtheit (nicht erfindungsgemäß) umfassend ein expandierbares Zwischenwirbelimplantat und ein Instrument zum Injizieren eines fluiden und aushärtbaren Mediums;
- Figur 6b: die Sachgesamtheit im zusammengefügten Zustand;
- Figur 7: eine Schnittansicht durch die Sachgesamtheit nach Figur 6;
- Figuren 8a, b, c: verschiedene Darstellungen einer Ausführungsform eines erfindungsgemäßen Zwischenwirbelimplantats und
- Figur 9: eine perspektivische Ansicht eines Zwischenwirbelimplantats (nicht erfindungsgemäß), welches mit einer Osteosynthesevorrichtung verbunden ist.

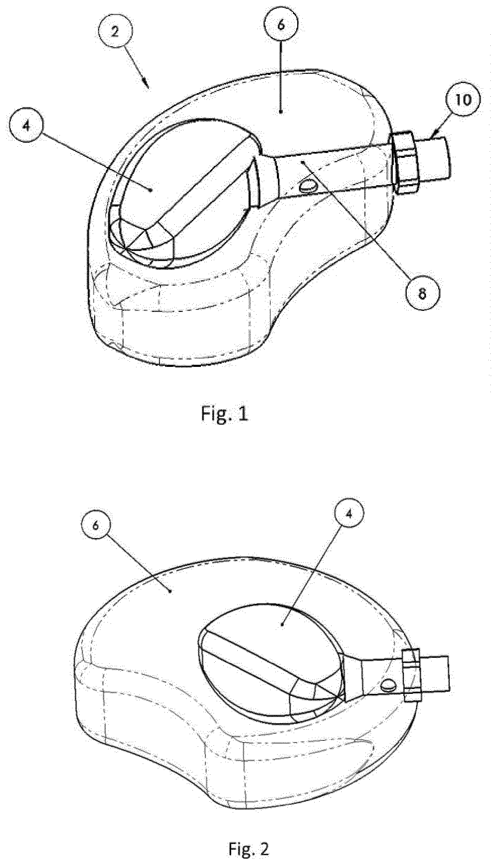

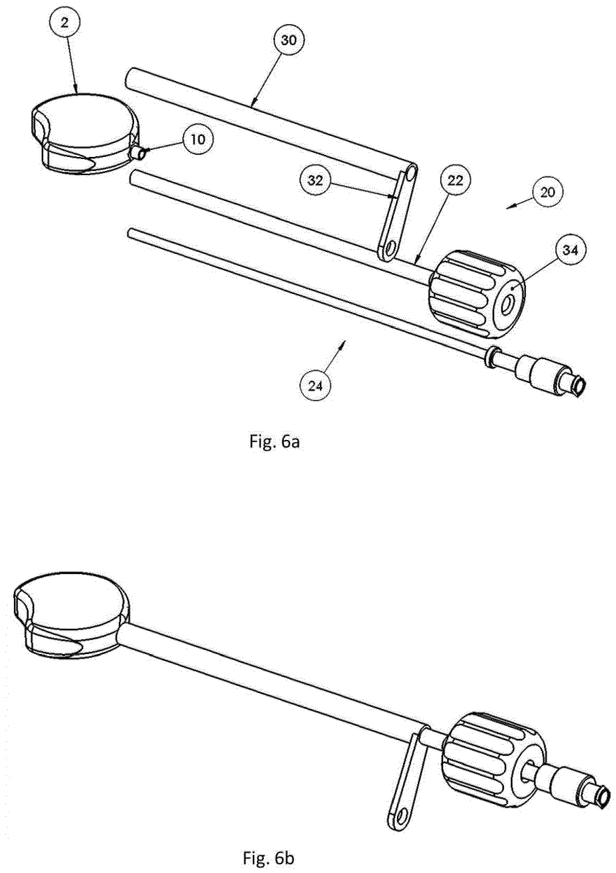

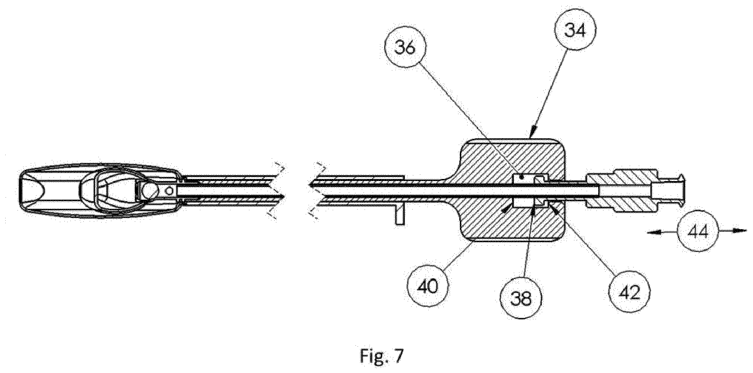

Figur 1 zeigt ein expandierbares Zwischenwirbelimplantat 2 für den Bandscheibenersatz, insbesondere im thorakalen und lumbalen Wirbelsäulenbereich. Es umfasst ein inneres Ballonelement 4 und ein äußeres Ballonelement 6, die jeweils mittels eines injizierbaren fluiden und aushärtbaren Mediums, insbesondere Knochenzement, füllbar sind und im befüllten Zustand ein Bandscheibenfach in einem Zwischenwirbelbereich distrahieren und als Bandscheibenersatz dienen sollen. Das innere Ballonelement 4 kommuniziert über ein leitungsbildendes Verbindungselement 8 mit einem in Figuren 6 und 7 dargestellten Instrument zum Injizieren eines fluiden und aushärtbaren Mediums. Das leitungsbildende Verbindungselement 8 führt nach außerhalb des äußeren Ballons 6 und weist dort eine Instrumentenanbindungseinrichtung 10 auf. Das innere Ballonelement 4 weist ungefähr die Form eines teilweise leicht abgeplatteten Ellipsoids auf, was aber rein beispielhaft ist.

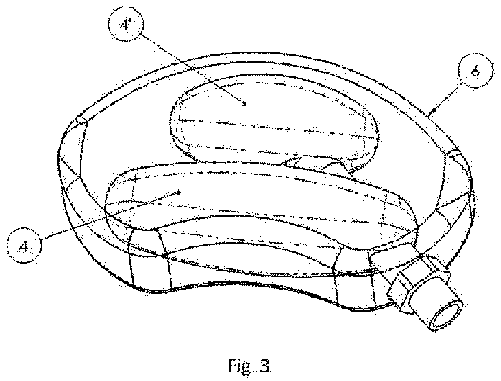

Das äußere Ballonelement 6 nimmt im injizierten, d. h. mit fluidem aushärtbarem Medium befüllten Zustand ungefähr die Form der zu ersetzenden Bandscheibe ein, wobei durch Injizieren des Mediums in das innere Ballonelement 4, aber auch in das äußere Ballonelement 6 eine parallele Bandscheibenfacherhöhung oder eine Bandscheibenfacherhöhung, die anterior oder posterior stärker ausgebildet ist, realisiert wird. Auf diese Weise kann ein Lordosewinkel oder ein Kyphosewinkel verändert werden. Dies kann durch Anordnung des inneren Ballonelements 4 an geeigneter Stelle innerhalb des äußeren Ballonelements 6 erreicht werden. Das Ballonelement 4 ist beispielsweise in Figur 1 anterior und in Figur 2 eher zentral positioniert. In Figur 3 sind zwei innere Ballonelemente 4, 4' dargestellt, von denen das eine 4 posterior und das andere 4' anterior innerhalb des äußeren Ballonelements 6 positioniert ist.

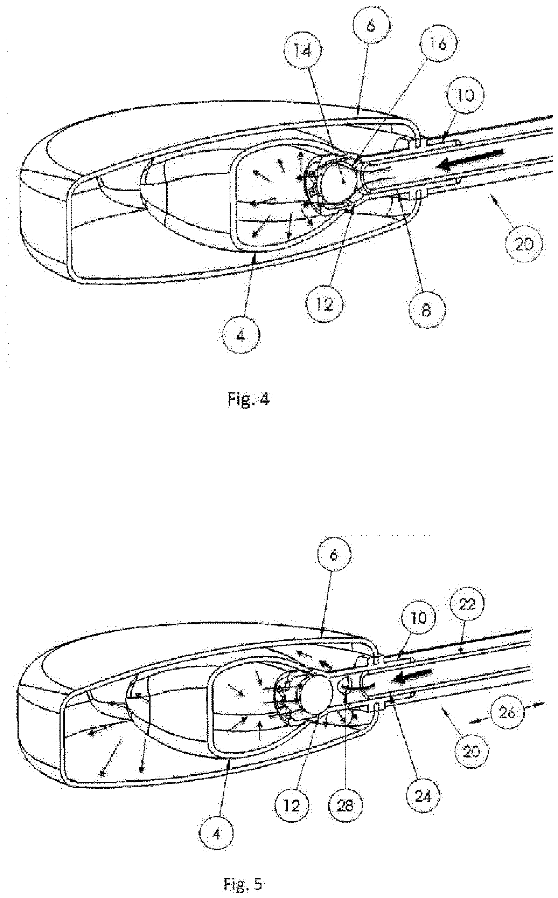

Die Figuren 4 und 5 verdeutlichen anhand der Pfeile die Befüllung oder Injizierung der Ballonelemente und damit die Distraktion des Bandscheibenfachs. Das leitungsbildende Verbindungselement 8 kommuniziert druckdicht mit dem Inneren des inneren Ballonelements 4 und führt außerdem druckdicht nach außerhalb des äußeren Ballonelements 6. Im Bereich der Anbindung an das innere Ballonelement 4 umfasst das Verbindungselement 8 ein Ventil 12, welches beispielhaft einen Ventilkörper 14 aufweist, der in Richtung auf das Innere des inneren Ballonelements 4 von einem Ventilsitz 16 abhebbar bzw. zum Verschließen gegen den Ventilsitz 16 anlegbar ist. Wenn in dem leitungsbildenden Verbindungselement 8 ein Fülldruck beim Injizieren des fluiden und aushärtbaren Mediums anliegt, so öffnet das Ventil 12 und das fluide aushärtbare Medium gelangt um den Ventilkörper 14 herum in das Innere des inneren Ballonelements 4. Hierdurch wird das innere Ballonelement 4 ausgefüllt und nimmt eine vorgegebene Vorzugsorientierung im Wesentlichen ein. Diese Vorzugsorientierung ergibt sich aus der Gestaltung des inneren Ballonelements 4 aus Flachmaterial bzw. aneinander gefügten Flachmaterialstücken.

Wird der Fülldruck in dem leitungsbildenden Verbindungselement 8 zurückgenommen, schließt das Ventil 12 aufgrund des im Inneren des inneren Ballonelements 4 herrschenden Drucks, was durch die Pfeile im Inneren des inneren Ballonelements 4 in Figur 5 angedeutet ist. Das Ventil 12 hält also den Druck innerhalb des inneren Ballonelements 4. Solang das Medium noch nicht ausgehärtet ist, wird die Bandscheibenfachhöhe von dem Ventil 12 selbständig gehalten. Eine Korrektur der Höhe oder des Lordose- oder Kyphosewinkels kann weiter schrittweise und insbesondere unter Röntgenkontrolle erfolgen, indem weiteres Medium in den inneren Ballon 4 eingeleitet werden kann.

In Figuren 4 und 5 ist ein in Figuren 6 und 7 dargestelltes Instrument 20 an die schon erwähnte Instrumentenanbindungseinrichtung oder -stelle 10 des leitungsbildenden Verbindungselements 8 angeschlossen. Das Instrument 20 umfasst eine Applikatorhülse 22 und eine darin innen längsverschieblich angeordnete Zementierungskanüle 24. In Figur 5 ist die innere Zementierungskanüle 24 von dem Ventil 12 weg in proximale Richtung 26 zurückgezogen. Auf diese Weise wird eine Durchgangsöffnung 28 in dem leitungsbildenden Verbindungselement 8 freigegeben, welche außerhalb des inneren Ballonelements 4 in das Innere des äußeren Ballonelements 6 mündet. Wird nun ein Injektionsdruck über die Zementierungskanüle 24 an das leitungsbildende Verbindungselement 8 gegeben, so tritt fluides Medium durch diese Durchgangsöffnung 28 hindurch in das Innere des äußeren Ballonelements 6, was in Figur 5 durch Pfeile angedeutet ist. Hierdurch wird das äußere Ballonelement 6 an die Bandscheibenfachkontur angepasst. Das innere Ballonelement 4 bleibt durch die Ventilwirkung weiterhin verschlossen. Sollte die Befüllung des äußeren Ballonelements 6 zu einem höheren Druck führen, so öffnet sich das Ventil 12 zum inneren Ballonelement 4 und das Medium fließt gleichzeitig auch in das innere Ballonelement 4, was zu einem Druckgleichgewicht zwischen beiden Ballonkammern führt.

Die Figuren 6 und 7 zeigen eine Sachgesamtheit aus einem erfindungsgemäßen Zwischenwirbelimplantat 2 und einem zum Befüllen der Ballonelemente geeigneten Instrument 20. Das Instrument 20 umfasst im beispielhaft dargestellten Fall die Applikatorhülse 22, die innere Zementierungskanüle 24 sowie eine Gegenhalterhülse 30 mit einem Halteschenkel 32. Die Applikatorhülse 22 umfasst weiter ein kopfförmiges Griffteil 34 mit einer im Inneren ausgebildeten Kammer 36. Im Bereich der Kammer 36 umfasst die innere Zementierungskanüle 24 einen radialen Vorsprung 38, der an einen distalen Anschlag 40 und an einen proximalen Anschlag 42 anlegbar ist, die auch die Kammer 36 begrenzen. Auf diese Weise ist die innere Zementierungskanüle 24 begrenzt in Längsrichtung 44 hin und her verschiebbar relativ zu der Applikatorhülse 22.

Durch dieses Verschieben der Zementierungskanüle 24 in Längsrichtung 44 kann die Öffnung 28 zum Inneren des äußeren Ballonelements 6 verschlossen oder freigegeben werden. Bei Fülldruckbeaufschlagung wird im verschlossenen Zustand der Durchgangsöffnung 28 das innere Ballonelement 4 befüllt und im freigegebenen Zustand das äußere Ballonelement 6 befüllt.

Durch geeignete Ausbildung des leitungsbildenden Verbindungselements 8 kann das innere Ballonelement 4 in der Längsrichtung 44 an unterschiedlicher Position innerhalb des äußeren Ballonelements 6 positioniert werden.

Es ist auch denkbar (nicht dargestellt), dass die Zementierungskanüle an ihrem distalen Ende verschlossen oder verschließbar ist und selbst wenigstens eine seitliche Austrittsöffnung für das zu injizierende fluide oder aushärtbare Medium aufweist. Auf diese Weise kann durch Drehen der Zementierungshülse innerhalb der Applikatorhülse eine fluchtende Anordnung der Öffnung in der Zementierungshülse mit der Durchgangsöffnung 28 in dem leitungsbildenden Verbindungselement 8 oder einer weiteren nicht dargestellten Öffnung im Inneren des ersten Ballonelements herbeigeführt werden, um das äußere oder das innere Ballonelement zu befüllen.

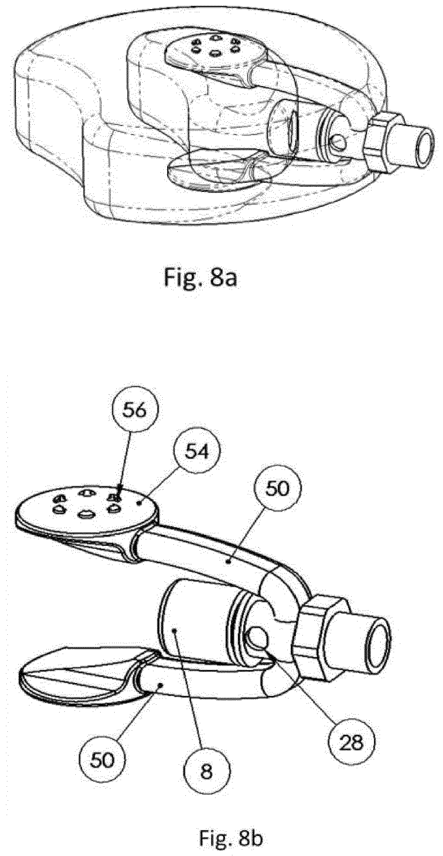

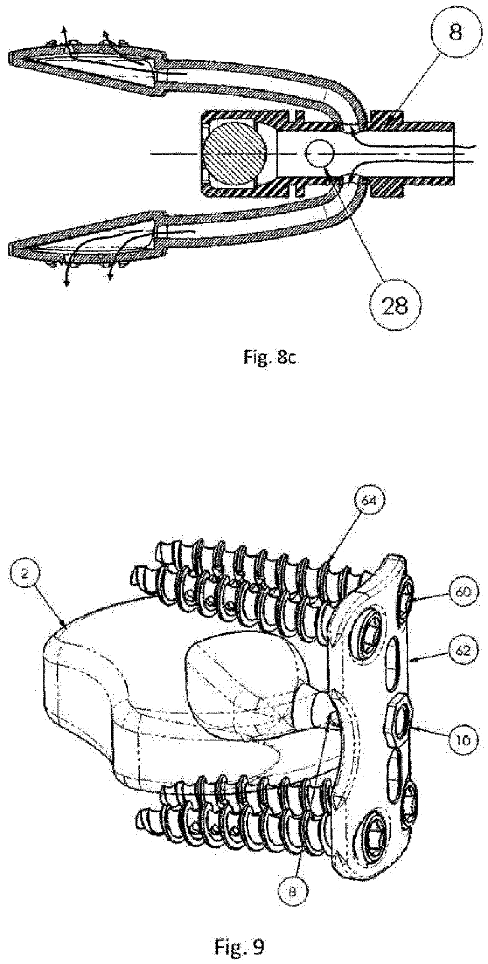

Die Figuren 8a bis c zeigen dass das leitungsbildende Verbindungselement 8 wenigstens eine zusätzliche Zuführleitung 50 für das injizierbare fluide und aushärtbare Medium umfasst, die in der oberen und/oder unteren Wandung des äußeren Ballonelements 6 nach außen mündet, so dass das injizierte Medium in angrenzende poröse Knochenbereiche eindringen kann. Im beispielhaften Fall mündet die zusätzliche Zuführleitung 50 in eine teller- oder scheibenförmige Erweiterung 54, die von innen oder von außen gegen eine Flachseite des äußeren Ballonelements 6 anliegt. An dieser teller- oder scheibenförmigen Erweiterung 54 sind mit dem Inneren der Erweiterung 54 und der Zuführleitung 50 kommunizierende spikeartige hohle Ansätze 56 ausgebildet, die von innen eine obere oder untere Wandung des äußeren Ballonelements 6 durchdringen können und sich in der Folge in knöcherne Deckplatten des darüber bzw. darunter liegenden Wirbelkörpers eindrücken können. Dies kann weiter dadurch unterstützt werden, dass das innere Ballonelement 4 direkt auf diese teller- oder scheibenförmige Erweiterung 54 von innen drückt. Somit kann in einer weit fortgeschrittenen osteoporotischen Ausgangssituation eine zusätzliche Wirbelkörperaugmentation erzielt werden. Dies hat den Vorteil, dass eine bessere Lastverteilung von den Wirbelkörpern auf das Zwischenwirbelimplantat erzielt werden kann. Des Weiteren können durch die Einleitung des aushärtbaren fluiden Mediums in den Knochen Wirbelkörperfrakturen oder eine Implantatsinterung vermindert oder sogar verhindert werden.

Figur 9 zeigt eine Halte- oder Tragverbindung zwischen einem erfindungsgemäßen Zwischenwirbelimplantat 2 und einer Osteosynthesevorrichtung 60. Die Osteosynthesevorrichtung bzw. eine Platte 62 der Osteosynthesevorrichtung 60 sind mit dem leitungsbildenden Verbindungselement 8 des Implantats durch eine stabile Tragverbindung gekoppelt. Somit ist auch die Instrumentenanbindungseinrichtung 10 des leitungsbildenden Verbindungselements 8 bei der Platte 62 ausgebildet. Die Platte 62 umfasst Öffnungen für insbesondere fenestrierte Osteosyntheseschrauben 64.

## Patentansprüche

1. Expandierbares Zwischenwirbelimplantat (2) für den Bandscheibenersatz, insbesondere im thorakalen und lumbalen Wirbelsäulenbereich, mit mindestens einem inneren Ballonelement (4) und einem äußeren Ballonelement (6), wobei das innere und das äußere Ballonelement jeweils für sich mittels eines injizierbaren fluiden und aushärtbaren Mediums füllbar sind, wobei das äußere Ballonelement (6) beim Einbringen in ein Bandscheibenfach in einem Zwischenwirbelbereich als Bandscheibenersatz ungefähr die Gestalt der zuvor entfernten Bandscheibe einnehmen soll, und eine obere und eine untere Wandung umfasst, mittels derer es im injizierten Zustand gegen eine das Bandscheibenfach oben und unten begrenzende Seite der angrenzenden Wirbelkörper anlegbar ist, und wobei das innere Ballonelement (4) eine anteriore oder posteriore oder parallele Bandscheibenfacherhöhung bewirkt, indem es im injizierten Zustand von innen gegen das äußere Ballonelement (6) mittelbar oder unmittelbar stützend anliegt, **dadurch gekennzeichnet,**
**dass** wenigstens eine zusätzliche Zuführleitung (50) für das injizierbare fluide und aushärtbare Medium vorgesehen ist, die in der oberen und/oder unteren Wandung des äußeren Ballonelements (6) nach außen mündet, so dass das injizierte Medium in angrenzende poröse Knochenbereiche eindringen kann.

2. Zwischenwirbelimplantat nach Anspruch 1, **dadurch gekennzeichnet, dass** die zusätzliche Zuführleitung (50) in eine teller- oder scheibenförmige Erweiterung (54) mündet, die von innen oder von außen gegen eine Flachseite des äußeren Ballonelements (6) anliegt.

3. Zwischenwirbelimplantat nach Anspruch 2, **dadurch gekennzeichnet, dass** an dieser teller- oder scheibenförmigen Erweiterung (54) mit dem Inneren der Erweiterung (54) und der Zuführleitung (50) kommunizierende spikeartige hohle Ansätze (56) ausgebildet sind, die in knöcherne Deckplatten des darüber bzw. darunterliegenden Wirbelkörpers eindrücken können.

4. Zwischenwirbelimplantat nach einem oder mehreren der vorstehenden Ansprüche, **dadurch gekennzeichnet, dass** von dem inneren Ballonelement (4) ein leitungsbildendes Verbindungselement(8) nach außerhalb des äußeren Ballonelements (6) führt, an welches ein Instrument (20) zum Injizieren des fluiden und aushärtbaren Mediums lösbar ankoppelbar ist.

5. Zwischenwirbelimplantat nach Anspruch 4, **dadurch gekennzeichnet, dass** das leitungsbildende Verbindungselement (8) zugleich die Zuführleitung (50) umfasst oder mit ihr kommuniziert.

6. Zwischenwirbelimplantat nach Anspruch 4 oder 5, **dadurch gekennzeichnet, dass** das leitungsbildende Verbindungselement (8) ein Ventil (12) umfasst, welches bei von außen anliegenden Injektionsdruck öffnet und bei Wegnahme des von außen anliegenden Injektionsdrucks schließt.

7. Zwischenwirbelimplantat nach einem oder mehreren der Ansprüche 4-6, **dadurch gekennzeichnet, dass** das leitungsbildende Verbindungselement (8) in einem Bereich zwischen einer Außenseite des inneren Ballonelements (4) und einer Innenseite der äußeren Ballonelements (6) eine Öffnung (28) zum Injizieren des fluiden und aushärtbaren Mediums in das Innere des äußeren Ballonelements aufweist und dass die Öffnung (28) zum Injizieren des fluiden und aushärtbaren Mediums in das äußere Ballonelement (6) beim Injizieren des fluiden und aushärtbaren Mediums in das innere Ballonelement verschließbar ist.

8. Zwischenwirbelimplantat nach einem oder mehreren der Ansprüche 4-7, **dadurch gekennzeichnet, dass** das leitungsbildende Verbindungselement (8) gegenüber dem äußeren Ballonelement (6) längenverstellbar ist, so dass das innere Ballonelement (4) nach anterior und posterior verlagerbar ist.

9. Zwischenwirbelimplantat nach einem oder mehreren der vorstehenden Ansprüche, **dadurch gekennzeichnet, dass** das äußere Ballonelement (6) außen eine Nano-, Mikro- oder Makrostrukturierung oder spikeartige Vorsprünge aufweist.

10. Expandierbares Zwischenwirbelimplantat (2) für den Bandscheibenersatz, insbesondere im thorakalen und lumbalen Wirbelsäulenbereich, mit einem einzigen Ballonelement (6), das mittels eines injizierbaren fluiden und aushärtbaren Mediums füllbar ist, wobei das Ballonelement (6) beim Einbringen in ein Bandscheibenfach in einem Zwischenwirbelbereich als Bandscheibenersatz ungefähr die Gestalt der zuvor entfernten Bandscheibe einnehmen soll, und eine obere und eine untere Wandung umfasst, mittels derer es im injizierten Zustand gegen eine das Bandscheibenfach oben und unten begrenzende Seite der angrenzenden Wirbelkörper anlegbar ist, wobei das Ballonelement (6) im injizierten Zustand eine anteriore oder posteriore oder parallele Bandscheibenfacherhöhung bewirkt, **dadurch gekennzeichnet, dass** wenigstens eine zusätzliche Zuführleitung (50) für das injizierbare fluide und aushärtbare Medium vorgesehen ist, die in der oberen und/oder unteren Wandung des Ballonelements (6) nach außen mündet, so dass das injizierte Medium in angrenzende poröse Knochenbereiche eindringen kann.

11. Zwischenwirbelimplantat nach Anspruch 10, **dadurch gekennzeichnet, dass** die zusätzliche Zuführleitung (50) in eine teller- oder scheibenförmige Erweiterung (54) mündet, die von innen oder von außen gegen eine Flachseite des äußeren Ballonelements (6) anliegt.

12. Zwischenwirbelimplantat nach Anspruch 10 oder 11, **dadurch gekennzeichnet, dass** an dieser teller- oder scheibenförmigen Erweiterung (54) mit dem Inneren der Erweiterung (54) und der Zuführleitung (50) kommunizierende spikeartige hohle Ansätze (56) ausgebildet sind, die in knöcherne Deckplatten des darüber bzw. darunterliegenden Wirbelkörpers eindrücken können

13. Sachgesamtheit aus einem expandierbaren Zwischenwirbelimplantat (2) nach einem oder mehreren der vorstehenden Ansprüche und einem Instrument (20) zum Injizieren des fluiden und aushärtbaren Mediums in das innere und das äußere Ballonelement, wobei das Instrument (20) eine äußere Hülse (22) umfasst, die an ein leitungsbildendes Verbindungselement (8) lösbar ankoppelbar ist, und eine innenliegende Kanüle(24), die in der äußeren Hülse (24) in einer Längsrichtung (44) verschieblich ist.

14. Sachgesamtheit nach Anspruch 13, **dadurch gekennzeichnet, dass** die Kanüle in der äußeren Hülse (24) in einer Längsrichtung (44) begrenzt verschieblich ist, indem sie wenigstens einen radialen Vorsprung (38) aufweist, der in einer von einem Griffelement (34) der äußeren Hülse (22) begrenzten Kammer (36) gegen einen Axialanschlag (40, 42) oder vorzugsweise in beiden Richtungen gegen je einen Axialanschlag (40,42) anlegbar ist.

15. Sachgesamtheit nach Anspruch 13 oder 14, **dadurch gekennzeichnet, dass** die Kanüle (24) in der äußeren Hülse (22) um eine Längsrichtung (44) drehbar ist und eine seitliche Fluidaustrittsöffnung aufweist, die durch Drehung in eine fluchtende Anordnung mit der Öffnung (28) in dem leitungsbildenden Verbindungselement (8) bringbar ist, so dass durch Drehung der Kanüle (24) eine Fluidkommunikation zum Infiltrieren des äußeren Ballonelements (6) herstellbar und blockierbar ist.

## Claims

1. Expandable intervertebral implant (2) for intervertebral disc replacement, in particular in the thoracic and lumbar vertebral column region, with at least one inner balloon element (4) and an outer balloon element (6), wherein the inner and the outer balloon elements can each be filled individually with an injectable, fluidic and hardenable medium, wherein on insertion into an intervertebral disc space in an intervertebral region as an intervertebral disc replacement, the outer balloon element (6) is to assume approximately the shape of the intervertebral disc previously removed, and comprises an upper and lower wall by means of which, in injected state, it can be laid against a side of the adjacent vertebral body delimiting the intervertebral disc space at the top and bottom, and wherein the inner balloon element (4) causes an anterior or posterior or parallel disc space height enlargement in that, in injected state, it lies directly or indirectly supportingly from the inside against the outer balloon element (6), **characterised in that**
at least one additional supply line (50) is provided for the injectable fluidic and hardenable medium, which opens to the outside in the upper and/or lower wall of the outer balloon element (6) so that the injectable medium can penetrate into adjacent porous bone regions.

2. Intervertebral implant according to claim 1, **characterised in that** the additional supply line (50) opens into a plate-like or disc-like widening (54) which lies from the inside or outside against the flat side of the outer balloon element (6).

3. Intervertebral implant according to claim 2, **characterised in that** spike-like hollow attachments (56) are formed on this plate-like or disc-like widening (54), which communicate with the interior of the widening (54) and of the supply line (50) and can press into bony cover plates of the vertebral body lying above or below.

4. Intervertebral implant according to one or more of the preceding claims, **characterised in that** a line-forming connecting element (8) leads from the inner balloon element (4) towards the outside of the outer balloon element (6), onto which an instrument (20) for injecting the fluidic and hardenable medium can be releasably coupled.

5. Intervertebral implant according to claim 4, **characterised in that** the line-forming connecting element (8) also comprises or communicates with the supply line (50).

6. Intervertebral implant according to claim 4 or 5, **characterised in that** the line-forming connecting element (8) comprises a valve (12) which opens under injection pressure applied from the outside and closes when the injection pressure applied from the outside is removed.

7. Intervertebral implant according to one or more of the claims 4 - 6, **characterised in that**, in a region between an outside of the inner balloon element (4) and an inside of the outer balloon element (6), the line-forming connecting element (8) has an opening (28) for injection of the fluidic and hardenable medium into the interior of the outer balloon element,
and that the opening (28) for injection of the fluidic and hardenable medium into the outer balloon element (6) can be closed on injection of the fluidic and hardenable medium into the inner balloon element.

8. Intervertebral implant according to one or more of claims 4 - 7, **characterised in that** the length of the line-forming connecting element (8) can be adjusted relative to the outer balloon element (6) so that the inner balloon element (4) is displaceable in the anterior and posterior directions.

9. Intervertebral implant according to one or more of the preceding claims, **characterised in that** the outer balloon element (6) has a nano-, micro- or macro-structuring or spike-like protrusions.

10. Expandable intervertebral implant (2) for intervertebral disc replacement, in particular in the thoracic and lumbar vertebral column region, with a single balloon element (6) which can be filled with an injectable, fluidic and hardenable medium, wherein on insertion into an intervertebral disc space in an intervertebral region as an intervertebral disc replacement, the balloon element (6) is to assume approximately the shape of the intervertebral disc previously removed, and comprises an upper and lower wall by means of which, in injected state, it can be laid against a side of the adjacent vertebral body delimiting the intervertebral disc space at the top and bottom, and wherein the balloon element (6) causes an anterior or posterior or parallel disc space height enlargement, **characterised in that** at least one additional supply line (50) is provided for the injectable fluidic and hardenable medium which opens towards the outside in the upper and/or lower wall of the balloon element (6) so that the injected medium can penetrate into adjacent porous bone regions.

11. Intervertebral implant according to claim 10, **characterised in that** the additional supply line (50) opens into a plate-like or disc-like widening (54) which lies from the inside or outside against the flat side of the outer balloon element (6).

12. Intervertebral implant according to claim 10 or 11, **characterised in that** spike-like hollow attachments (56) are formed on this plate-like or disc-like widening (54), which communicate with the interior of the widening (54) and of the supply line (50), and can press into bony cover plates of the vertebral body lying above or below.

13. System comprising an expandable intervertebral implant (2) according to one or more of the preceding claims, and an instrument (20) for injecting the fluidic and hardenable medium into the inner and outer balloon element, wherein the instrument (20) comprises an outer sleeve (22) which can be coupled releasably to a line-forming connecting element (8), and an inner cannula (24) which is displaceable in a longitudinal direction (44) in the outer sleeve (24).

14. System according to claim 13, **characterised in that** the cannula has limited displaceability in a longitudinal direction (44) in the outer sleeve (24) **in that** it has at least one radial protrusion (38) which can be placed in a chamber (36), delimited by a handle element (34) of the outer sleeve (22), against an axial stop (40, 42) or preferably can lie against a respective axial stop (40, 42) in both directions.

15. System according to claim 13 or 14, **characterised in that** the cannula (24) is rotatable about a longitudinal direction (44) in the outer sleeve (22) and has a lateral fluid-outlet opening which can be brought by rotation into an aligned arrangement with the opening (28) in the line-forming connecting element (8), so that by rotation of the cannula (24), a fluidic communication for infiltration of the outer balloon element (6) can be created and blocked.

## Revendications

1. Implant intervertébral expansible (2) pour le remplacement d'un disque intervertébral, en particulier dans la zone thoracique et lombaire de la colonne vertébrale, avec au moins un élément ballonnet intérieur (4) et un élément ballonnet extérieur (6), dans lequel le ballonnet intérieur et le ballonnet extérieur peuvent être remplis chacun au moyen d'un milieu fluide et durcissable injectable, dans lequel l'élément ballonnet extérieur (6), lorsqu'il est introduit dans un espace de disque intervertébral dans une zone intervertébrale en tant que remplacement de disque intervertébral, vise à adopter approximativement la forme du disque intervertébral préalablement retiré, et comprend une paroi supérieure et une paroi inférieure, au moyen desquelles il peut être placé dans l'état injecté contre une face, délimitant en haut et en bas l'espace de disque intervertébral, des corps vertébraux adjacents, et dans lequel l'élément ballonnet intérieur (4) provoque une augmentation de l'espace de disque intervertébral antérieure ou postérieure ou parallèle, du fait que, dans l'état injecté, il s'applique de l'intérieur indirectement ou directement en appui contre l'élément ballonnet extérieur (6), **caractérisé en ce qu'**au moins une conduite d'amenée (50) supplémentaire pour le milieu fluide et durcissable injectable est prévue, qui débouche vers l'extérieur dans la paroi supérieure et/ou inférieure de l'élément ballonnet extérieur (6), de sorte que le milieu injecté peut pénétrer dans des zones osseuses poreuses adjacentes.

2. Implant intervertébral selon la revendication 1, **caractérisé en ce que** la conduite d'amenée (50) supplémentaire débouche dans une extension (54) en forme d'assiette ou de disque, qui s'applique de l'intérieur ou de l'extérieur contre une face plate de l'élément ballonnet extérieur (6).

3. Implant intervertébral selon la revendication 2, **caractérisé en ce que** des parties saillantes (56) creuses du type clou communiquant avec l'intérieur de l'extension (54) et de la conduite d'amenée (50), qui peuvent s'enfoncer dans des plaques de recouvrement osseuses du corps vertébral situé dessus ou dessous, sont réalisées sur cette extension (54) en forme d'assiette ou de disque.

4. Implant intervertébral selon l'une ou plusieurs des revendications précédentes, **caractérisé en ce qu'**un élément de liaison (8) formant conduite va de l'élément ballonnet intérieur (4) vers l'extérieur de l'élément ballonnet extérieur (6), auquel un instrument (20) destiné à injecter le milieu fluide et durcissable peut être accouplé de manière détachable.

5. Implant intervertébral selon la revendication 4, **caractérisé en ce que** l'élément de liaison (8) formant conduite comprend en même temps la conduite d'amenée (50) ou communique avec elle.

6. Implant intervertébral selon la revendication 4 ou 5, **caractérisé en ce que** l'élément de liaison (8) formant conduite comprend une soupape (12), laquelle s'ouvre lorsqu'une pression d'injection est appliquée de l'extérieur et se ferme lorsque la pression d'injection appliquée de l'extérieur est retirée.

7. Implant intervertébral selon l'une ou plusieurs des revendications 4-6, **caractérisé en ce que** l'élément de liaison (8) formant conduite présente dans une zone entre une face extérieure de l'élément ballonnet intérieur (4) et une face intérieure de l'élément ballonnet extérieur (6) une ouverture (28) destinée à l'injection du milieu fluide et durcissable à l'intérieur de l'élément ballonnet extérieur
et que l'ouverture (28) destinée à l'injection du milieu fluide et durcissable dans l'élément ballonnet extérieur (6) peut être fermée lors de l'injection du milieu fluide et durcissable dans l'élément ballonnet intérieur.

8. Implant intervertébral selon l'une ou plusieurs des revendications 4-7, **caractérisé en ce que** l'élément de liaison (8) formant conduite est réglable en longueur par rapport à l'élément ballonnet extérieur (6), de sorte que l'élément ballonnet intérieur (4) peut être déplacé de manière antérieure et postérieure.

9. Implant intervertébral selon l'une ou plusieurs des revendications précédentes, **caractérisé en ce que** l'élément ballonnet extérieur (6) présente à l'extérieur une nano-, micro- ou macro-structuration ou des saillies du type clou.

10. Implant intervertébral expansible (2) pour le remplacement d'un disque vertébral, en particulier dans la zone thoracique et lombaire de la colonne vertébrale, avec un seul élément ballonnet (6), qui peut être rempli au moyen d'un milieu fluide et durcissable injectable, dans lequel l'élément ballonnet (6), lorsqu'il est introduit dans un espace de disque intervertébral dans une zone intervertébrale en tant que remplacement de disque intervertébral, vise à adopter approximativement la forme du disque intervertébral préalablement retiré, et comprend une paroi supérieure et une paroi inférieure, au moyen desquelles il peut être placé dans l'état injecté contre une face, délimitant en haut et en bas l'espace de disque intervertébral, des corps vertébraux adjacents, dans lequel l'élément ballonnet (6) provoque dans l'état injecté une augmentation de l'espace de disque intervertébral antérieure ou postérieure ou parallèle, **caractérisé en ce qu'**au moins une conduite d'amenée (50) supplémentaire pour le milieu fluide et durcissable injectable est prévue, qui débouche vers l'extérieur dans la paroi supérieure et/ou inférieure de l'élément ballonnet (6), de sorte que le milieu injecté peut pénétrer dans des zones osseuses poreuses adjacentes.

11. Implant intervertébral selon la revendication 10, **caractérisé en ce que** la conduite d'amenée (50) supplémentaire débouche dans une extension (54) en forme d'assiette ou de disque, qui s'applique de l'intérieur ou de l'extérieur contre une face plate de l'élément ballonnet extérieur (6).

12. Implant intervertébral selon la revendication 10 ou 11, **caractérisé en ce que** des parties saillantes (56) creuses du type clou communiquant avec l'intérieur de l'extension (54) et de la conduite d'amenée (50), qui peuvent s'enfoncer dans des plaques de recouvrement osseuses du corps vertébral situé dessus ou dessous, sont réalisées sur cette extension (54) en forme d'assiette ou de disque.

13. Ensemble composé d'un implant intervertébral expansible (2) selon l'une ou plusieurs des revendications précédentes et d'un instrument (20) destiné à injecter le milieu fluide et durcissable dans l'élément ballonnet intérieur et l'élément ballonnet extérieur, dans lequel l'instrument (20) comprend un manchon extérieur (22), qui peut être accouplé de manière détachable à un élément de liaison (8) formant conduite, et une canule (24) intérieure, qui est mobile dans le manchon extérieur (24) dans une direction longitudinale (44).

14. Ensemble selon la revendication 13, **caractérisé en ce que** la canule est mobile de façon limitée dans le manchon extérieur (24) dans une direction longitudinale (44), du fait qu'elle présente au moins une saillie radiale (38), qui, dans une chambre (36) délimitée par un élément de préhension (34) du manchon extérieur (22), peut être appuyée contre une butée axiale (40, 42) ou de préférence dans les deux directions contre respectivement une butée axiale (40, 42).

15. Ensemble selon la revendication 13 ou 14, **caractérisé en ce que** la canule (24) dans le manchon extérieur (22) peut être amenée en rotation autour d'une direction longitudinale (44) et présente une ouverture de sortie de fluide latérale, qui peut être amenée par rotation dans un agencement an affleurement avec l'ouverture (28) dans l'élément de liaison (8) formant conduite, de sorte que, par rotation de la canule (24), une communication fluidique destinée à infiltrer l'élément ballonnet extérieur (6) peut être établie et bloquée.
